# EUROPEAN PATENT SPECIFICATION

(11) **EP 3 190 898 B1**
(45) Date of publication and mention of the grant of the patent: **24.01.2018**
(21) Application number: 15760471.1
(22) Date of filing: 10.09.2015
(51) Int. Cl.: A23D 9/007, A61K 31/122

(54) **UBIDECARENONE COMPOSITION**
UBIDECARENONZUSAMMENSETZUNG
COMPOSITION D'UBIDÉCARÉNONE

(30) Priority: 10.09.2014 EP 14184328
(43) Date of publication of application: 19.07.2017
(73) Proprietor: Pharma Nord ApS, 6500 Vojens (DK)
(72) Inventor: MOESGAARD, Sven, DK-6051 Almind (DK); PAULIN, Helge Schousen, DK-6580 Vamdrup (DK)
(74) Representative: COPA Copenhagen Patents
(86) International application number: PCT/EP2015/070743
(87) International publication number: WO 2016/038150

(56) References cited:
- EP-A1- 1 475 363
- EP-A1- 1 591 020
- EP-A1- 2 402 007
- KR-A- 20080 100 575
- US-A1- 2010 168 249
- "Coenzyme=q(10) compsn. for soft capsules - comprising soln. in neutral soil, e.g. soybean oil, in the presence of surfactant, e.g. polyoxyethylene mono:stearate", WPI / THOMSON,, vol. 1980, no. 31, 20 June 1980 (1980-06-20), XP002485958,

## Description

### FIELD OF THE INVENTION

The present invention relates to a composition comprising ubidecarenone, wherein composition includes a mixture of low-temperature melting fat or oil and high temperature melting fat or oil.

### BACKGROUND OF THE INVENTION

Ubidecarenone (also known as coenzyme Q10 (CoQ10) or ubiquinone), is a 1,4-benzoquinone, where Q refers to the quinone chemical group, and 10 refers to the number of isoprenyl chemical subunits in its tail.

Ubidecarenone is present in most eukaryotic cells, primarily in the mitochondria. It is a component of the electron transport chain and participates in aerobic cellular respiration, generating energy in the form of ATP. There are three redox states of CoQ10: fully oxidized (ubiquinone), semiquinone (ubisemiquinone), and fully reduced (ubiquinol). The capacity of this molecule to exist in a completely oxidized form and a completely reduced form enables it to perform its functions in the electron transport chain, and as an antioxidant, respectively. The highest ubidecarenone concentrations are found in heart, liver and kidney.

Ubidecarenone is contained in food and is also endogenously produced in the body. It works both as an antioxidant and as an energy transporter in the inner membrane of mitochondria. As a medicine, it is used for heart failure patients and as a food supplement it is consumed for nutritional purposes.

Ubidecarenone is a highly lipophilic substance that is practically insoluble in water. It is a large molecule (863 g/mol) which has low bioavailability when ingested. It is soluble in ethanol, hexane and fats or oils. The melting point of Q10 is 48°C. Exposure of Q10 to higher temperatures for longer time turns ubidecarenone into ubichrominol-9 or other undesired degradation products.

Powder of ubidecarenone can be dissolved in heated fat or oil. Conventional compositions of Q10 for human consumption therefore usually comprise fat or oil in which Q10 is solubilized or partly solubilized depending on the concentration of Q10. Thus, softgel capsules comprising ubidecarenone and fat or oil are well-known in the art. Prior art EP1 591 020 relates to a method for improving the stability of coenzyme Q10 by emulsifying the said component with fatty acid ester of polyhydric alcohol. Upon cooling to below its melting point, oversaturated solutions of Q10 in traditional fat or oil compositions spontaneously form amorphous crystals typically 50 x 50 µm and varying from 10 - 200 µm. The ubidecarenone present in these compositions is in a form that impairs the bioavailability of Q10. Traditional softgel formulations consist of 10 - 200 mg ubidecarenone and 300 - 1000 mg fat or oil in a softgel capsule. The majority of the Q10 present in such capsules is present as amorphous crystals. Accordingly, the delivery of Q10 is impaired by the very low solubility in water and the resulting low dissolution rate after ingestion of the capsules containing mainly amorphous crystals of ubidecarenone. Consequently, Q10 poses significant problems in terms of delivery to the human or animal body. Undissolved Q10 will not enter the human or animal body where it exerts its beneficial effects.

Several possibilities for increasing the dissolution rate of Q10 *in vivo* have been explored, most of which focus on providing delivery of Q10 as micronized particles. However, the substantially spherical particles resulting from micronization are undesirable as they are difficult to work with and tend to reaggregate during formulation and storage. Further, solubilizing micronized Q10 in fat or oil will result in re-appearance of amorphous crystals when Q10 recrystallizes in the formulations during storage. Thus, there is a need in the art for new compositions, preferably compositions providing increased dissolution *in vivo*, and thereby providing improved bioavailability when ingested by human or animal subjects.
The crystal formation of Q10 in softgel capsules can be observed by microscope, measured by nuclear magnetic resonance (NMR) or directly measured after separating the content of a capsule by high gravity centrifugation. Further, the bioavailability of ubidecarenone can be measured by supplementing humans in controlled trials with different types of capsules. In vitro testing of release profiles with standard methods may further simulate bioavailability.

### SUMMARY OF THE INVENTION

During experiments leading to the present invention, it was surprisingly discovered that new compositions of ubidecarenone facilitate the formation and preservation of new crystal forms of ubidecarenone comprising a plurality of elongated parts, the elongated parts extending or protruding from a core region. These Q10 crystal forms are characterised in having a visual appearance as an assembly of spikes growing outwards from a central region. The surface-to-volume-ratios of these new crystals were observed to be comparable or even higher than previously known micronized Q10 particles.

This discovery made possible the production of compositions comprising discrete particles of Q10, each particle providing a high bioavailability. These compositions provide improved bioavailability of ubidecarenone. Further, the compositions provide extended shelf-life of ubidecarenone in bioavailable form.

Accordingly, the invention relates to a composition comprising ubidecarenone solubilized in an oil matrix, wherein the oil matrix comprises a solution of a low-temperature melting soybean oil, and at least one high-temperature melting soybean fat or oil, said low-temperature melting fat or oil having a melting point that is at least 10°C lower than the melting temperature of one or more of the high-temperature melting fats or oils.

The new composition may facilitate the formation and preservation of the new crystal form of ubidecarenone as can be seen in the experimental part of the present application.

Essentially, the inventive compositions comprising fat or oil having a low melting point and oil or fat having a high melting point, may be used for solubilizing powdered Q10 particles into the composition at high temperature followed by cooling of the resulting compositions, preferably to a temperature below the melting point of Q10, even more preferably to a temperature below 37°C.

It was found essential that the melting temperature of the different oil constituents (high vs. low temperature melting) differed by at least 10 °C.

In a highly preferred embodiment, the compositions according to the invention comprise an oil mixture consisting of 4 parts of oil per 1 part Q10 (w/w), wherein the oil consists of 75% soybean oil (the low-temperature-melting fat or oil) and 25% hydrogenated soybean oil (the high-temperature-melting fat or oil).

The invention further relates to softgel capsules comprising the new crystal form of Q10.

### DEFINITIONS

"High-temperature-melting fat or oil" according to the invention means, in its broadest sense, a triglyceride that has a melting point (temperature) that is at least 10°C higher than the melting point (temperature) of the low-temperature melting fats or oils. Preferably, however, "High-temperature-melting fat or oil" according to the invention has a melting point (temperature) of at least 30°C, as determined by appropriate methods described in Ph.Eur. Alternative methods are described in DAB (deutsches Arzneibuch) or British Pharmacopoeia. The "high-temperature melting fat or oil" according to the invention may be a natural triglyceride or a triglyceride which has been hydrogenated or partly hydrogenated to increase the melting temperature. Alternatively, it may be a natural hydrogenated or partly hydrogenated triglyceride or a natural non-hydrogenated triglyceride, preferably, with a melting point of at least 30°C.

"Low-temperature melting fat or oil" according to the invention means, in its broadest sense, a triglyceride that has a melting point (temperature) that is at least 10°C lower than the melting point (temperature) of the high temperature melting fats or oils according to the invention. Preferably, however, "Low-temperature-melting fat or oil" according to the invention means a triglyceride with a melting point below 15°C determined by methods described in Ph.Eur. Alternative methods are described in DAB (deutsches Arzneibuch) or British Pharmacopoeia.

Unless otherwise stated, parts, concentrations or ratios mentioned in the present description are based on weight (w/w).

### DETAILED DESCRIPTION OF THE INVENTION

During the development work, a new type of crystals of ubidecarenone has been discovered. The new crystal form of ubidecarenone has improved properties in that it provides an increased dissolution rate in water and improved bioavailability *in vivo*.

The present invention, however, is seen as the provision of a composition, which may facilitate and maintain the formation of the new crystal form of ubidecarenone. Thus, it was surprisingly discovered that the new and improved crystal forms of ubidecarenone can be maintained in stable form in compositions according to the invention, said compositions comprising a specific mixture of low-temperature melting fat or oils, and high-temperature melting fats or oils, even after re-heating and subsequent cooling.

The new form of ubidecarenone is produced by melting ubidecarenone powder in the characteristic fat/oil matrix and mixing the ingredients according to a special procedure at controlled temperatures and time intervals, as seen e.g. in the examples.

As seen in the below examples, *in vitro* tests in form of dissolution studies show that the new needle-shaped ubidecarenone crystals dissolve more rapidly and completely at human body temperature of 37°C than the conventional rhomboid crystals.

The amount of ubidecarenone in the solution is dependent on the temperature. At room temperature (20°C), as much as 80% of ubidecarenone in a softgel capsule according to the invention may be found to be in crystals, such as stars or needle-shaped crystals.

As seen in the experimental section, *in vitro* and *in vivo* experiments have documented that ubidecarenone from softgel capsules according to the invention is better absorbed by the human body than ubidecarenone from softgel capsules which contain amorphous crystals.

### Compositions according to the invention

The compositions according to the invention comprise ubidecarenone, and a fat or oil solution wherein ubidecarenone is dispersed or solubilized.

These compositions are characterised in being able to contain dispersed ubidecarenone crystals at room temperature (20°), and solubilized ubidecarenone at a temperature of (37°) or above.

The inventive compositions comprise an oil component having a low melting temperature and an oil component having a high temperature melting point. It was found that an increased difference in melting temperature between the low-temperature melting fat or oil and the lowest melting temperature of the high-temperature melting fats or oils resulted in improved characteristics of the inventive compositions, in that less high-temperature melting oil was needed.

It is preferred, that the compositions according to the invention comprise an oil solution consisting of 4 parts of oil per 1 part Q10 (w/w), wherein the oil consists of 75% soybean oil and 25% hydrogenated soybean oil.

In a most preferred embodiment, the invention relates to a composition comprising ubidecarenone solubilized in an oil matrix, wherein the oil matrix comprises an oil solution consisting of approximately 4 parts oil per 1 part Q10 (w/w), wherein the oil consists of 75% soybean oil, 20% hydrogenated soybean oil with a melting point 32°C and 5% hydrogenated soybean oil with a melting point 41°C.

By "approximately" is meant that the relative parts may deviate by up to 10%.

In a particularly preferred embodiment of the invention, the composition comprises partly hydrogenated soybean oil with a melting point of 32°C as a first high-temperature melting fat or oil, partly hydrogenated soybean oil with a melting point of 41°C as a second high-temperature melting fat or oil, and soybean oil with a melting point of - 16°C as the low-temperature melting fat or oil.

In a highly preferred embodiment of the invention, the compositions comprise 100 mg ubidecarenone, 297 mg soybean oil, 79 mg hydrogenated soybean oil with melting point 32°C and 20 mg hydrogenated soybean oil with melting point 41°C.

Further, as part of the present invention, research has been performed in order to find the minimum capsule size (the minimal composition) that may contain the largest amount of needle-shaped crystals of ubidecarenone and still go quickly into solution *in vivo* at a temperature of 37°C *in vivo*. The optimal ratio was found to be approximately 1:4 between ubidecarenone and fat or oil matrix.

Accordingly, in a preferred embodiment, the compositions according to the invention comprise 1 part Q10 per 3 - 5 parts fat or oil. Even more preferred, the compositions according to the invention comprise 1 part Q10 per 4 parts fat or oil.

Thus, the invention relates to a composition, wherein the ratio of fat and/or oil to ubidecarenone per weight is approximately 4:1.

The compositions according to the invention may also contain further ingredients. As an appropriate example of further ingredients is antioxidants, such as e.g. vitamin E.

A highly preferred composition according to the invention is a softgel capsule containing a composition according to the invention, the composition comprising (per 500 mg composition) approximately 100 mg ubidecarenone, 297 mg soybean oil, 79 mg hydrogenated soybean oil with a melting point of 32°C, 20 mg hydrogenated soybean oil with a melting point of 41°C, and 4 mg Vitamin E 1 IU/mg.

A typical softgel capsule shell is composed of gelatine, glycerol, water and colouring agents.

In one embodiment, the compositions according to the present invention are intended for use as a medicine.

In another embodiment, the compositions according to the present invention are intended for use as a food or food supplement.

### EXAMPLES

### Example 1

A softgel capsule containing ubidecarenone in an oil solution according to the invention was produced as follows:
74250 g (74.25 kg) soybean oil was loaded into a low speed mixer under continuous stirring with low speed mixer set at 10 rpm and at a pre-set temperature of 47°C. To this soybean oil was added 24750 g partly hydrogenated soybean oil with a melting point of 32°C, 5000 g partly hydrogenated soybean oil with a melting point of 41°C and 1000 g vitamin E under continuous stirring. An oil solution was formed.

25000 g ubidecarenone powder was added to the solution under continuous stirring.

After addition of ubidecarenone powder the temperature was adjusted to 43°C. The solution was kept under constant stirring at 43°C for 60 minutes. The solution was visually checked and observed to be a clear solution without any visible floating material. If the temperature is too low or there is visible precipitate, then stirring and heating should continue.

The resulting solution was cooled and filled into softgel capsules each containing:

| | |
|---|---|
| Ubidecarenone | 100 mg |
| Soybean oil | 297 mg |
| Hydrogenated soybean oil with melting point 32°C | 79 mg |
| Hydrogenated soybean oil with melting point 41°C | 20 mg |
| Vitamin E 1 IU/mg | 4 mg |
| **Total** | **500 mg** |

**Capsule shell:**

| | |
|---|---|
| Gelatine | 110 - 148 mg |
| Glycerol | 47 - 64 mg |
| Colour | 2 mg |
| Purified water | 12 - 32 mg |
| **Grand total** | **approx. 700 mg** |

Cooling ubidecarenone according to this invention from the manufacturing temperature to room temperature at different rates was not found to have a significant effect on the ubidecarenone composition.

### Example 2

Softgel capsules were produced by mixing the ingredients of Example 1 other than ubidecarenone at 47° followed by cooling to room temperature and adding ubidecarenone. Hereafter, the resulting dispersion was loaded into softgel capsules, each containing the same ingredients and amounts thereof as the softgel capsules according to Example 1.

The content of the softgel capsules produced according to Example 1 and the softgel capsules produced according to Example 2 were analysed by microscopy. The results confirmed the presence of an oil solution in the softgel capsules produced according to Example 1. The softgel capsules produced according to Example 2 contained amorphous crystals.

When heating the softgel capsules in a water bath to a temperature of 37°C, it was further observed (by NMR) that the contents of the capsules according to Example 1 become liquid and that substantially all ubidecarenone crystals are solubilized.

This was not the case for the capsules according to Example 2.

However, processing the compositions of example 2, according to the procedure described in example 1 result in the production of capsules with the same characteristics as the capsules of example 1.

### Example 3 (Reference example)

104.000 g soybean oil was loaded into a low speed mixer under continuous stirring with a low-speed mixer set at 10 rpm and at a pre-set temperature of 47°C. To this soybean oil was added 1.000 g vitamin E under continuous stirring.

25.000 g ubidecarenone powder with amorphous ubidecarenone crystal structure was added to the blend under continuous stirring.

After addition of ubidecarenone powder, the temperature was adjusted to 43°C. The solution was kept under constant stirring at 43°C for 60 minutes. The solution was visually checked and observed to be a clear solution without any visible crystals. If the temperature is too low or there are visible crystals, then stirring and heating should continue. The resulting solution was filled into softgel capsules as described in Example 1.

The contents of the softgel capsules produced were analysed by microscopy and the absence of ubidecarenone crystals was confirmed.

Further, heating the compositions of the capsules according to example 3, to a temperature where Q10 solubilises, did not result in the production of capsules with the same characteristics as the softgel capsules produced according to Example 1, i.e. comprising the new crystal form of Q10. Processing the compositions of example 3, according to the procedure described in example 1 do not result in the production of capsules with the same characteristics as the capsules of example 1.

### Example 4

### In vitro test

The contents of three 100 mg ubidecarenone softgel capsules produced according to example 1 were emptied into an Eppendorf tube. As a reference, the contents of three 100 mg ubidecarenone softgel capsules produced according to Example 2 were emptied into an Eppendorf tube and analysed in parallel.

The two Eppendorf tubes were placed in a water bath at 31°C, and the temperature was then raised gradually to 38°C. When the temperature reached 37°C, the Eppendorf tubes were turned gently and carefully and repeatedly turned as the temperature increased to 38°C. At 38°C, the Eppendorf tubes were removed from the water bath and centrifuged at 14,000 rpm for 6 minutes at 37°C.

The Eppendorf tube containing the ubidecarenone compositions according to the invention produced only insignificant precipitate, whereas the reference compositions gave considerable amounts of crystallized precipitate that is not bioavailable.

### Example 5

### In vivo test

The purpose of this example was to compare the ubidecarenone crystalline structure, i.e. amorphous versus needle-shaped crystals, in softgel capsules, and the influence thereof on the absorption of ubidecarenone in humans.

### Method

1 softgel capsule produced according to Example 1 or a reference softgel capsule produced according to Example 2 was given to five healthy male volunteers aged 23 - 33 years in a randomised, double-blind, single-dose crossover trial with a one-week interval between treatments. A blood sample was obtained from each person in the morning and immediately after the single capsule was taken with water.

After treatment, the subjects fasted for 5 hours, after which a second blood sample was obtained. The procedure was repeated with random crossover one week later.

Serum was obtained from the blood samples, and was kept frozen until analysed for ubidecarenone. All samples were analyzed in one run by a standard HPLC method.

### Results

### Capsules according to Example 1:

| ***Serum ubidecarenone concentration (mg*/*l)*** | | | |
|---|---|---|---|
| **Test person** | **Baseline** | **5 hour value** | **Difference** |
| 1 | 0.92 | 1.02 | 0.10 |
| 2 | 0.91 | 0.95 | 0.04 |
| 3 | 0.54 | 0.57 | 0.03 |
| 4 | 0.67 | 0.73 | 0.06 |
| 5 | 0.81 | 0.91 | 0.10 |

Median (and average) increase from baseline = 0.06 (0.07).

### Capsules according to Example 2:

| ***Serum ubidecarenone concentration (mg*/*l)*** | | | |
|---|---|---|---|
| **Test person** | **Baseline** | **5 hour value** | **Difference** |
| 1 | 1.15 | 1.17 | 0.02 |
| 2 | 0.82 | 0.86 | 0.04 |
| 3 | 0.58 | 0.59 | 0.01 |
| 4 | 0.73 | 0.65 | - 0.08 |
| 5 | 1.28 | 1.18 | - 0.10 |

Median (and average) increase from baseline = 0.01 (-0.02).

The increase in serum concentration of ubidecarenone from the single dose of capsules containing ubidecarenone according to the invention was in 5 cases out of 5 greater than or equal to the reference that contains amorphous crystals of ubidecarenone:

| **Test** | **Amorphous** | **Cone-shaped** | **Difference** |
|---|---|---|---|
| 1 | 0.02 | 0.10 | 0.08 |
| 2 | 0.04 | 0.04 | 0.00 |
| 3 | 0.01 | 0.03 | 0.02 |
| 4 | -0.08 | 0.06 | 0.14 |
| 5 | -0.10 | 0.10 | 0.20 |
| Median | 0.01 | 0.06 | 0.08 |

The median increase of plasma ubidecarenone found in this study with 5 fasting persons at 5 hours after a single dose of softgel capsule with 100 mg ubidecarenone was 0.06 mg/l for softgel capsules that contain ubidecarenone according to the invention, and 0.01 mg/l in the reference amorphous formulation.

The study confirms a significant difference in absorption of the two forms (p=0.0625) in favour of the crystal form of ubidecarenone according to the invention. Hence, this example indicates an *in vivo* difference in the rate of absorption of ubidecarenone formulations between cone-shaped crystals and amorphous crystals.

## Claims

1. A composition comprising ubidecarenone solubilized in an oil matrix, wherein the oil matrix comprises an oil solution consisting of approximately 4 parts oil per 1 part Q10 (w/w), wherein the oil consists of 75% soybean oil, 20% hydrogenated soybean oil with a melting point 32°C and 5% hydrogenated soybean oil with a melting point 41°C.

2. The composition according to claim 1, comprising 100 mg ubidecarenone, 297 mg soybean oil, 79 mg hydrogenated soybean oil with melting point 32°C and 20 mg hydrogenated soybean oil with melting point 41°C.

3. The composition according to claim 1 or 2, comprising vitamin E.

4. Softgel capsule comprising the composition according to any of claims 1-3.

5. Composition according to any one of claims 1-3 for use as a medicine.

6. Composition according to any one of claims 1-3 for use as a food or food supplement.

## Patentansprüche

1. Zusammensetzung, umfassend Ubidecarenon, gelöst in einer Ölmatrix, wobei die Ölmatrix eine Öllösung umfasst, die aus etwa 4 Teilen Öl je 1 Teil Q10 (Gew./Gew.) besteht, wobei das Öl zu 75 % aus Sojaöl, zu 20 % aus hydriertem Sojaöl mit einem Schmelzpunkt von 32 °C und zu 5 % aus hydriertem Sojaöl mit einem Schmelzpunkt von 41 °C besteht.

2. Zusammensetzung nach Anspruch 1, umfassend 100 mg Ubidecarenon, 297 mg Sojaöl, 79 mg hydriertes Sojaöl mit einem Schmelzpunkt von 32 °C und 20 mg hydriertes Sojaöl mit einem Schmelzpunkt von 41 °C.

3. Zusammensetzung nach Anspruch 1 oder 2, umfassend Vitamin E.

4. Weichgelkapsel umfassend die Zusammensetzung nach einem der Ansprüche 1-3

5. Zusammensetzung nach einem der Ansprüche 1-3 zur Verwendung als Arzneimittel.

6. Zusammensetzung nach einem der Ansprüche 1-3 zur Verwendung als Nahrungsmittel oder Nahrungsergänzungsmittel.

## Revendications

1. Composition comprenant de l'ubidécarénone solubilisée dans une matrice d'huile, dans laquelle la matrice d'huile comprend une solution d'huile constituée d'environ 4 parties d'huile pour 1 partie de Q10 (p/p), dans laquelle l'huile est constituée de 75 % d'huile de soja, de 20 % d'huile de soja hydrogénée présentant un point de fusion de 32 °C et de 5 % d'huile de soja hydrogénée présentant un point de fusion de 41 °C.

2. Composition selon la revendication 1, comprenant 100 mg d'ubidécarénone, 297 mg d'huile de soja, 79 mg d'huile de soja hydrogénée présentant un point de fusion de 32 °C et 20 mg d'huile de soja hydrogénée présentant un point de fusion de 41 °C.

3. Composition selon la revendication 1 ou 2, comprenant de la vitamine E.

4. Capsule molle comprenant la composition selon l'une quelconque des revendications 1 à 3.

5. Composition selon l'une quelconque des revendications 1 à 3 destinée à être utilisée en tant que médicament.

6. Composition selon l'une quelconque des revendications 1 à 3 destinée à être utilisée en tant qu'aliment ou complément alimentaire.
